# EUROPÄISCHE PATENTANMELDUNG

(11) **EP 2 742 975 A1**
(43) Veröffentlichungstag der Anmeldung: **18.06.2014**
(21) Anmeldenummer: 12197355.6
(22) Anmeldetag: 14.12.2012
(51) Int. Cl.: A61Q 3/02, A61K 8/04, A61K 8/25, A61K 8/34, A61K 8/55, A61K 8/81, A61K 8/85, A61K 8/87, A61K 8/86

(54) **Nagellack**

(71) Anmelder: Faber- Castell AG, 90546 Stein (DE)
(72) Erfinder: Lugert, Dr. Gerhard, 90431 Nürnberg (DE); Kaul, Dr. Wolfgang, 91560 Heilsbronn (DE); Appel, Tatiana, 90522 Oberasbach (DE)
(74) Vertreter: Meissner, Bolte & Partner GbR

(57) **Zusammenfassung**

Die Erfindung betrifft einen Nagellack, mit einer Viskosität von weniger als 50 mPas Brookfield LV, DV III Ultra Rheometer, Spindel: 40, RPM: 10, 25°C) und folgender Zusammensetzung:
- 20% bis 85% einer wässrigen Kunststoffdispersion als Filmbildner, mit einem Festkörperanteil von 20% bis 60%
- 5% bis 20% eines Feuchthaltemittels,
- 1% bis 10% eines Farbmittels, das zumindest zum Teil aus einem Farbpigment gebildet ist,
- 0,1% bis 0,5% eines Schichtsilikats
- 0% bis 5% eines Tensids,
- 0% bis 5% eines Additivs
- Rest: Wasser.

## Beschreibung

Die Erfindung betrifft einen Nagellack, der mit einem ein kapillares Auftragselement und einen Flüssigkeitsspeicher aufweisenden kapillaren Auftragsgerät auf einen Finger- oder Fußnagel applizierbar ist. Ein Nagellack der sich für eine solche Applikation eignet muss eine vergleichsweise niedrige Viskosität von weniger als 50 mPas (Brookfield LV, DV III Ultra Rheometer, Spindel: 40, RPM: 10, 25°C) aufweisen, damit er mit Hilfe eines kapillaren, beispielsweise faserartigen, von Poren durchsetzten Auftragselements applizierbar ist. Nagellacke der in Rede stehenden, beispielsweise der aus DE 199 59 093 A1 bekannten Art, enthalten eine wässrige Polymerdispersion, wobei diese bzw. deren Polymeranteil in erster Linie zur Bildung eines Films auf der Nageloberfläche dient. Zur Färbung ist wenigstens ein Farbpigment vorhanden. Des Weiteren ist dem Nagellack ein Feuchthaltemittel zugesetzt, welches ein Eintrocknen des Nagellacks in dem kapillaren Auftragselement verhindert oder zumindest verzögert und außerdem die Filmbildung durch die Polymerdispersion unterstützt. Schließlich enthält der bekannte Nagellack gegebenenfalls Additive wie Konservierungsmittel oder pflegende Substanzen.

Nagellacke der vorliegenden Art neigen bei länger andauernder Lagerung zur Farbveränderung, was auf eine Sedimentation von Farbpigmenten zurückzuführen ist. Dabei kommt es ganz allgemein zu einer Verringerung der in der wässrigen Phase dispergierten Pigmente, somit zu einer Verringerung der Farbintensität. Im Falle von Gemischen von Farbpigmenten mit unterschiedlicher Farbe können Farbverschiebungen dadurch entstehen, dass die einzelnen Farbpigmente mit unterschiedlichen Geschwindigkeiten sedimentieren.

Ein weiteres Problem liegt darin, dass viele Dispergiermittel zu einer Erhöhung der Viskosität über die o.g. Viskositätsgrenze hinaus führen, was den auf Kapillarkräfte beruhenden Transport des Nagellacks durch die Poren eines kapillaren Auftragselements des Auftragsgeräts hindurch behindern würde. Problematisch ist schließlich noch, dass das Dispergiermittel die Filmbildung und die Qualität des entstehenden Lackfilms auf der Nageloberfläche negativ beeinflussen kann.

Aufgabe der Erfindung ist es, einen Nagellack vorzuschlagen, der hinsichtlich der vorgenannten Nachteile Abhilfe schafft.

Diese Aufgabe wird durch einen Nagellack gemäß Anspruch 1 gelöst. Ein erfindungsgemäßer Nagellack weist zunächst eine Viskosität von weniger als 50 mPas (Brookfield LV, DV III Ultra Rheometer, Spindel: 40, RPM: 10, 25°C) auf, was eine zwingende Voraussetzung dafür ist, dass der Lack mit einem ein kapillares Auftragselement aufweisenden Auftragsgerät applizierbar ist. Der Lack setzt sich zusammen aus 20% bis 85% einer wässrigen Kunststoffdispersion als Filmbildner, 5% bis 20% eines Feuchthaltemittels, 1% bis 10% eines zumindest teilweise aus Farbpigmenten gebildeten Farbmittels, 0,1% bis 0,5% eines Schichtsilikats, 0% bis 5%, vorzugsweise 0,5% bis 3% eines Additivs, und 0% bis 5%, vorzugsweise 0,5% bis 3% eines Tensids, wobei als Rest bzw. ad 100% Wasser enthalten ist.

Die genannten Inhaltstoffe können in Einzahl oder Mehrzahl vorhanden sein. So kann beispielsweise nur ein einziges Farbpigment oder eine Mischung verschiedener Farbpigmente vorhanden sein. Neben einem oder mehreren Farbpigmenten kann auch ein oder mehrere wasserlösliche Farbstoffe vorhanden sein.

Die Farbpigmente können in Form einer Pigmentzubereitung, etwa einer pastenartigen wässrigen Dispersion in den Nagellack eingebracht werden. Es ist aber ebenso möglich, die Pigmente in Pulverform zuzusetzen und die so erhaltene Vorstufe des Nagellacks in einer Kugelmühle oder einer vergleichbaren Vorrichtung zu behandeln, um die Farbpigmente zu dispergieren.

Alle in dem Nagellack enthaltenen Partikel, insbesondere die Farbpigmente sind kapillargängig, d.h. sie weisen eine Größe auf, die kleiner ist als die lichte Weite der Poren des Auftragselements, welches beispielsweise dochtartig ausgebildet ist und beispielsweise aus einem faserartigen, also aus einer Vielzahl von Fasern bestehenden Material oder aus einem Kunststoff-Sintermaterial gebildet ist. Während dies bei den verwendeten Kunststoffdispersionen bzw. den darin enthaltenden Kunststoffpartikeln mit Partikelgrößen im Nanometerbereich von Haus aus der Fall ist, weisen die Farbpigmente und das vorzugsweise in Pulverform zugesetzte Schichtsilikat Korngrößen von weniger als 20 µm auf. Im Falle der Farbpigmente liegt beispielsweise eine Korngrößenverteilung von D90 < 10µm vor.

Es hat sich überraschenderweise gezeigt, dass durch das Schichtsilikat, welches in erster Linie die Funktion eines Dispergiermittels hat, eine Lagerstabilität des Nagellacks von mindestens 6 Wochen gewährleistet ist, wobei dies unabhängig von der der Art der verwendeten Farbpigmente ist. Im Falle von Pigmentmischungen, ist somit eine Veränderung des ursprünglichen Mischungsverhältnisses der Pigmente durch unterschiedliche Sedimentation verhindert. Weiterhin war überraschend, dass das Schichtsilikat, obwohl es in Wasser quillt, die Viskosität des Nagellacks nicht oder nur in unerheblichem Ausmaß erhöht, so dass die Herstellung von Nagellacken mit einer unter dem in Rede stehenden Viskositätsgrenzwert von 50 mPas Brookfield LV, DV III Ultra Rheometer, Spindel: 40, RPM: 10, 25°C) problemlos möglich ist. Schließlich war noch überraschend, dass praktisch keine negativen Wechselwirkungen mit dem Filmbildner auftreten, etwa die Filmbildung behindert wird.

Die oben genannten vorteilhaften Eigenschaften und Wirkungen in besonderem Maße gewährleistende Schichtsilikate sind Lithium-Magnesium-Natrium-Silikate. Die Schichtsilikate werden der Ausgangsmischung der Lack-Bestandteile zweckmäßigerweise in Pulverform zugesetzt. Um eine feine Verteilung bzw. eine Dispersion des Schichtsilikats zu fördern, kann organisches Polyphospat als Additiv beigemengt werden, wobei dessen Anteil - bezogen auf den Gehalt des Schichtsilikats - 2% bis 25% beträgt. Das Polyphosphat ist zweckmäßigerweise mit dem Schichtsilikat zu einer Zubereitung vermengt, welche dem Nagellack bzw. der Ausgangsmischung der Lackbestandteile zugesetzt wird.

Die in einem Nagellack eingesetzte Kunststoffdispersion muss gut an der Nageloberfläche haften und einen dünnen, widerstandsfähigen und glatten Film bilden. Oft liegt eine Situation vor, bei der ein Kunststoff bzw. eine daraus hergestellte Kunststoffdispersion zwar gut an der Nageloberfläche haftet, dünne und glatte Filme bildet, diese aber keinen oder keinen zufriedenstellenden Glanz aufweisen. Wenn jedoch, wie dies bei einer bevorzugten Ausführungsvariante der Fall ist, der Nagellack ein Tensid mit einem Anteil von 0,5% bis 3% enthält, wobei ein anionisches Tensid, insbesondere ein Alkoxyalkan, etwa ein Polyalkylenglykolether, bevorzugt ist, ergeben sich Filme mit gutem Glanz. Die besten Ergebnisse hinsichtlich der Glanzwirkung werden mit einem Polyoxyethylen-(20)-oleylether erreicht.

Als Filmbildner des Nagellacks finden Kunststoffdispersionen Verwendung, die wenigstens ein Polymer aus der Gruppe Polyacrylate, Polyurethane und Polyester oder entsprechende Co-Polymere bzw. Hybridpolymere enthalten. Die Kunststoffdispersionen weisen einen Feststoff- bzw. Kunststoffanteil von 20% bis 60% auf. Ein Problem bei Filmbildnern für den in Rede stehenden Einsatzweck ist, dass die damit erzeugten Filme nach der Applikation eine oft Minuten lang anhaltende Klebrigkeit aufweisen und u.a. aus dem genannten Grund nur langsam trocknen. Anders ist dies, wenn der Nagellack als Filmbildner eine Kunststoffdispersion auf Basis eines aliphatischen Urethan-Acrylat-Hybridpolymers, insbesondere eines Urethan-2-Polymethyl-Methacrylat- Hybridpolymers aufweist. Ein solcher Nagellack zeigt nach der Applikation keine Klebrigkeit und ist bereits nach etwa 10 Sekunden vollständig getrocknet. Nachteilig bei den genannten Kunststoffdispersionen ist allerdings, dass sie relativ matt erscheinende Filme bilden. Diesbezüglich kann jedoch durch eine besonders bevorzugte Kombination aus den in Rede stehenden Kunststoffdispersionen und einem Tensid der weiter oben genannten Art Abhilfe geschaffen werden, d.h. mit einem entsprechend modifizierten Nagellack lassen sich nicht klebrige, schnell trocknende und glänzende Filme erzeugen.

Additive für den Nagellack sind vor allem Konservierungsmittel wie beispielsweise Phenoxyethanol und Caprylyl-Glykcol, Fungizide oder pflegende Substanzen.

In der untenstehenden Tabelle sind vier erfindungsgemäße Rezepturen A bis D sowie eine Vergleichsrezeptur V aufgeführt. Die entsprechenden Nagellacke wurden auf Fingernägel von Testpersonen appliziert und hinsichtlich der Gleichmäßigkeit, der Antrockenzeit, der Klebrigkeit nach der Applikation und des Glanzes der Nagellack-Filme untersucht. Außerdem wurde eine vorgegebene Menge der Nagellacke in einem Behälter 6 Wochen lang bei einer Temperatur von 20°C gelagert, danach auf eine Unterlage der o.g. Art appliziert und ein Farbvergleich mit Filmen vorgenommen, die mit nicht gelagertem, also frisch hergestellten Nagellacken, erzeugt wurden. Die Ergebnisse der Tests sind ebenfalls der untenstehenden Tabelle entnehmbar. Im Vergleich zum Nagellack V zeigen die Nagellacke B bis D keine klebrige Konsistenz und trocknen wesentlich schneller ab, was auf die als Filmbildner enthaltene Dispersion eines aliphatischen Urethan-Acrylat-Hybridpolymers, in den Ausführungsbeispielen eine Urethan-2-Polymethyl-Methacrylat- Hybridpolymer-Dispersion, zurückzuführen ist. Die damit erzeugten Filme sind von Haus aus matt, was in abgeschwächter Form auch für den Film gemäß Rezeptur V zutrifft. Durch Zugabe eines Tensids (Polyoxyethylen-(20)-oleylether) zum Nagellack werden jedoch Filme mit hohem Glanz erzeugt (A, C und D).

Bei den Rezepturen A und D, welche ein vorzugsweise in Pulverform zugesetztes Schichtsilikat enthalten, ist eine auf Sedimentationseffekte beruhende Farbänderung nach einer Lagerung von 6 Wochen nicht zu erkennen.

| | **V** | **A** | **B** | **C** | **D** |
|---|---|---|---|---|---|
| Feuchthaltemittel: Ethanol | 10,0% | 10,0% | 10,0% | 10,0% | 10,0% |
| Konservierung: Dermosoft octiol **¹⁾** | 0,5% | 0,5% | 0,5% | 0,5% | 0,5% |
| Konservierung: Phenoxyethanol | 0,5% | 0,5% | 0,5% | 0,5% | 0,5% |
| Wasser, dem. | 39,0% | 37,8% | 39,0% | 38,0% | 37,8% |
| Tensid: BRIJ-O20 ss-BR **²⁾** | | 1,0% | | 1,0% | 1,0% |
| Schichtsilikat: Laponite XLS **³⁾** | | 0,2% | | | 0,2% |
| Filmbildner: Syntran 5760 PE302-7 **⁴⁾** | 40,0% | 40,0% | | | |
| Filmbildner: Hybridur 875 5) | | | 40,0% | 40,0% | 40,0% |
| Filmbildner: Ammonium-Acrylat-Copolymer | | | | | |
| Farbpigment: Cosmenyl Carmin **⁶⁾** | 8,0% | 8,0% | 8,0% | 8,0% | 8,0% |
| Cosmenyl schwarz **⁶⁾** | 2,0% | 2,0% | 2,0% | 2,0% | 2,0% |
| | 100,0% | 100,0% | 100,0% | 100,0% | 100,0% |
| | | | | | |

| **Testergebnis** | | | | | |
|---|---|---|---|---|---|
| | | | | | |
| Gleichmäßiger Film | | **-** | **#** | **+** | **++** |
| Antrockenzeit | - ~60s | - ~60s | + ~10s | + ~10s | + ~10s |
| Klebrigkeit nach Applikation | - ja | ++ ja | ++ nein | ++ nein | ++ nein |
| Glanz | **+** | **++** | **#** matt | **++** | **++** |
| Farbänderung nach Lagerung | **-** | **++** | **-** | **-** | **++** |

| | | | | | |
|---|---|---|---|---|---|
| Legende zur Tabelle: ++ sehr gut / + gut / # akzeptabel / - schlecht | | | | | |

Ein weiteres Beispiel für Farbpigmente sind die unter dem Handelsnamen Covarine, etwa des Typs W, von der Firma Sensient, F-95310 Saint Quen L'Aumone.

Alle Prozent-Angaben in der Tabelle und an sonstiger Stelle sind Gewichtsprozente.

### Hersteller:

1) Dr. Straetmans Chemische Produkte GmbH, D-22143 Hamburg;
2) Croda GmbH, D-41334 Nettetal Kaldenkirchen;
3) Rockwood Clay Additives GmbH, D-85368 Moosburg;
4) Interpolymer GmbH, D-67454 Hassloch Synthran = Styrol/Acrylat/Ammonium/Methacrylat-Copolymer;
5) Air Products and Chemicals Inc., Allentown, USA,
6) Cariant, D-65926-Frankfurt.

## Patentansprüche

1. Nagellack, mit einer Viskosität von weniger als 50 mPas (Brookfield LV, DV III Ultra Rheometer, Spindel: 40, RPM: 10, 25°C) und folgender Zusammensetzung:
- 20% bis 85% einer wässrigen Kunststoffdispersion als Filmbildner, mit einem Festkörperanteil von 20% bis 60%,
- 5% bis 20% eines Feuchthaltemittels,
- 1% bis 10% eines Farbmittels, das zumindest zum Teil aus einem Farbpigment gebildet ist,
- 0,1% bis 0,5% eines Schichtsilikats
- 0% bis 5% eines Tensids,
- 0% bis 5% eines Additivs
- Rest: Wasser.

2. Nagellack nach Anspruch 1, **dadurch gekennzeichnet, dass** 0,5% bis 3% eines Tensids enthalten sind.

3. Nagellack nach Anspruch 1 oder 2, **dadurch gekennzeichnet, dass** 0,5% bis 3% eines Additivs enthalten sind.

4. Nagellack nach einem der vorhergehenden Ansprüche, **dadurch gekennzeichnet, dass** das Schichtsilikat ein Lithium-Magnesium-Natrium-Silikat ist.

5. Nagellack nach Anspruch 4, **dadurch gekennzeichnet, dass** als Additiv ein organisches Polyphospat enthalten ist.

6. Nagellack nach Anspruch 5, **dadurch gekennzeichnet, dass** der Anteil des Polyphosphats 2% bis 10% des Anteils des Schichtsilikats beträgt.

7. Nagellack nach einem der vorhergehenden Ansprüche, **gekennzeichnet durch** eine wässrige Kunststoffdispersion, die wenigstens ein Polymer aus der Gruppe Polyacrylate, Polyurethane und Polyester enthält.

8. Nagellack nach Anspruch 7, **dadurch gekennzeichnet, dass** der Filmbildner eine Dispersion eines aliphatischen Urethan-Acrylat-Hybridpolymers ist.

9. Nagellack nach Anspruch 8, **dadurch gekennzeichnet** durch eine Urethan-2-Polymethyl-Methacrylat- Hybridpolymer-Dispersion.

10. Nagellack nach einem der vorhergehenden Ansprüche, **gekennzeichnet durch** ein nichtionisches Tensid.

11. Nagellack nach Anspruch 10, **dadurch gekennzeichnet, dass** das Tensid ein Alkoxyalkan ist.

12. Nagellack nach Anspruch 11, **dadurch gekennzeichnet, dass** das Tensid ein Polyalkylenglykolether ist.

13. Nagellack nach Anspruch 12, **gekennzeichnet durch** Polyoxyethylen-(20)-oleylether als Tensid.

14. Nagellack nach einem der vorhergehenden Ansprüche, **dadurch gekennzeichnet, dass** eine wässrige Pigmentpräparation mit einem Feststoffgehalt von 20% bis 60% enthalten ist.

15. Nagellack nach einem der vorhergehenden Ansprüche, **dadurch gekennzeichnet, dass** das Farbmittel einen wasserlöslichen Farbstoff umfasst.
